# EUROPEAN PATENT SPECIFICATION

(11) **EP 3 979 978 B1**
(45) Date of publication and mention of the grant of the patent: **24.09.2025**
(21) Application number: 20729972.8
(22) Date of filing: 26.05.2020
(51) Int. Cl.: A61K 8/34, A61P 17/10, A61Q 17/00, A61Q 19/10, A61K 31/045, A61K 31/05, A61P 17/02, A61P 31/02

(54) **USE OF TOPICAL COMPOSITION FOR BALANCING MICROBIOTA OF SKIN**
VERWENDUNG EINER TOPISCHEN ZUSAMMENSETZUNG ZUM AUSGLEICH DER MIKROBIOTA VON HAUT
UTILISATION D'UNE COMPOSITION TOPIQUE D'ÉQUILIBRAGE DU MICROBIOTE DE LA PEAU

(30) Priority: 06.06.2019 WO PCT/CN2019/090267; 10.07.2019 EP 19185501
(43) Date of publication of application: 13.04.2022
(73) Proprietor: Unilever IP Holdings B.V., 6708 WH Wageningen (NL); UNILEVER GLOBAL IP LIMITED, Wirral Merseyside CH62 4ZD (GB)
(72) Inventor: CHU, Chung-Ching, Shanghai, 200335 (CN); PU, Mingming, Shanghai, 200335 (CN); XU, Yining, Shanghai, 200335 (CN)
(74) Representative: James, Helen Sarah
(86) International application number: PCT/EP2020/064525
(87) International publication number: WO 2020/244962

(56) References cited:
- WO-A1-2010/046238
- WO-A1-2018/166758
- RAMAN A ET AL: "Antimicrobial effects of tea-tree oil and its major components on Staphylococcus aureus, Staph. epidermidis and Propionibacterium acnes", LETTERS IN APPLIED MICROBIOLOGY, OXFORD, GB, vol. 21, 1 January 1995 (1995-01-01), pages 242 - 245, XP002190828, ISSN: 1472-765X
- "A facewash containing specific plant-derived monoterpenes reducedPropionibacterium acnesburden and improved acne symptoms ED - Lim Henry W", JOURNAL OF THE AMERICAN ACADEMY OF DERMATOLOGY, MOSBY, INC, US, vol. 79, no. 3, 2 August 2018 (2018-08-02), XP085434231, ISSN: 0190-9622, DOI: 10.1016/J.JAAD.2018.05.095

## Description

### Field of the invention

The present invention relates to a method of balancing microbiota of amenable skin.

### Background of the invention

The skin is the largest organ of human body. It protects our body from external factors such as the environment, pollution and microbes. Our skin harbours a diverse range of microorganisms which live as a community and function as a component of skin biology. Due to the advent of next-generation sequencing technologies, we now know that the microbiome or microbiota of human skin is highly dependent on biophysical characteristics and chemical constitution of the skin. For example, sebaceous sites are dominated by *Cutibacterium sp.*, whereas moist areas harbour *Staphylococcus* and *Corynebacterium* species. Any adverse alteration in the microbiome of our skin could lead to conditions e.g., dysbiosis, that may need at least cosmetic treatment as such seen in the case of acne, dry skin and dandruff.

Several skin-derived lipids, including free fatty acids (FFAs) in sebum and sphingoid bases produced by the hydrolysis of ceramides of epidermal keratinocytes, and peptides, such as psoriasin, defensins and cathelicidin exhibit antimicrobial properties. These antimicrobial molecules naturally exist on the skin's surface forming an integral part of skin's defense or immunity that functions to balance skin's microbiome ecology. (Fischer et al.; Biochim Biophys Acta; 2014, Vol. 1841, Iss. 3, p. 319-322).

Acne vulgaris, a skin disorder common among adolescents which affects self-image and confidence of at least some of them. It is known that microbiome dysbiosis, increased sebum production, follicular hyperkeratinisation and inflammation are the most common contributing factors. *Cutibacterium acnes* (*C. acnes*, formerly known as *Propionibacterium acnes*) has long been considered a factor for acne. Recent studies reveal that acne is associated with an altered microbiota involving multiple taxa, as evidenced by an increase in the number of certain microbes and a concomitant decrease in the number of some others. Such an imbalance often manifests itself as a dysbiotic condition like acne and dandruff. Other such dysbiotic conditions include atopic dermatitis and dry skin. To some extent, such conditions can be managed by a cosmetic treatment, i.e. a non-therapeutic treatment, by the application of topical compositions that contain one or more active ingredients.

The microbiome of skin can be balanced e.g. by reducing the overload microbial load or microbial count of skin but by selectively reducing some while selectively increasing the count of some others.

In some cases, balancing the microbiota of skin refers to selectively increasing the microbial count of good microbes and/or reducing the same count of bad microbes. Good microbes are said to provide benefit to their host through e.g., competing with bad microbes for available nutrients and through secretion of good metabolites.

WO2018/050056 A1 (P&G) discloses a composition comprising a zinc compound, a biocompatible surfactant, and a lipid. for selectively increasing the diversity of the skin microbiota of a subject with an amenable skin condition which includes healthy skin and skin exhibiting atopic dermatitis (with or without lesions), skin dysbiosis and/or acne.

WO2018/049557 A1 (P&G) discloses a composition comprising a zinc compound (e.g., a zinc ionophore such as zinc pyrithione), a biocompatible surfactant (e.g., a mild surfactant such as laureth(n) sulfate (SLEnS)) and a lipid for selectively increasing the diversity of the skin microbiota of a subject with an amenable skin condition which includes healthy skin and skin exhibiting atopic dermatitis (with or without lesions), skin dysbiosis and/or acne.

WO19086327A1 (Unilever) discloses a new use of niacinamide or its analogue and precursors for balancing microbiome of skin, especially the scalp, against microorganisms. "A facewash containing specific plant-derived monoterpenes reduced Propionibacterium acnes burden and improved acne symptoms ED - Lim Henry W"(JOURNAL OF THE AMERICAN ACADEMY OF DERMATOLOGY, MOSBY, INC, US, vol. 79, no. 3, 2 August 2018 (2018-08-02), ISSN: 0190-9622, DOI: 10.1016/J.JAAD.2018.05.095) describes the treatment of acne using a facewash twice daily for a four week period. Facewash compositions comprising thymol and terpineol are disclosed (Abstract 7495). Baseline studies establish that in acne subjects P. acnes and S. epidermis are more abundant than in healthy skin. Where the acne is treated with the facewash the levels of the P.acnes and S. epidermis drop concurrently with the reduction in acne counts.

### Summary of the invention

In accordance with a first aspect disclosed is use of thymol and terpineol in a topical composition for balancing microbiota of healthy non-dysbiotic skin, where balancing means selectively reducing microbial count of at least one genus of microbes belong to *Staphylococcus or Cutibacterium,* while selectively increasing microbial count of at least one genus of microbes belong to *Streptoccocus*, *Moraxella or Deinococcus .*

In accordance with a second aspect disclosed is a method of balancing microbiota of healthy non-dysbiotic skin comprising a step of applying thereto thymol and terpineol in a topical composition, where balancing means selectively reducing microbial count of at least one genus of microbes belong to *Staphylococcus or Cutibacterium* while selectively increasing microbial count of at least one genus of microbes belong to *Streptoccocus, Moraxella or Deinococcus.*

In accordance with a third aspect disclosed is a topical composition comprising thymol and terpineol for use in balancing microbiota of dysbiotic skin which is dry skin or has at least one of acne, atopic dermatitis, dandruff, melasma or is pollution-exposed skin, where balancing means selectively reducing microbial count of at least one genus of harmful microbes belong to *Staphylococcus or Cutibacterium*, while selectively increasing microbial count of at least one genus of microbes belong to *Streptoccocus, Moraxella or Deinococcus.*

### Detailed description of the invention

Any feature of one aspect of the present invention may be utilized in any other aspect of the invention. The word "comprising" is intended to mean "including" but not necessarily "consisting of" or "composed of". In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "x to y" are understood to include x and y. When for a specific feature multiple preferred ranges are described in the format "x to y", it is understood that all ranges combining the different endpoints are also contemplated. Unless specified otherwise, amounts as used herein are expressed in percentage by weight based on total weight of the composition and is abbreviated as "wt%". The use of any and all examples or exemplary language e.g. "such as" provided herein is intended merely to better illuminate the invention and does not in any way limit the scope of the invention otherwise claimed.

The term balancing means selectively reducing microbial count of at least one genus of microbes considered to be harmful or of at least one genus of microbes that exhibit growth which is not representation of a normal healthy skin, while selectively increasing microbial count of at least one genus of microbes considered to be beneficial or of at least one genus of microbes whose numbers have reduced which is also not representation of a normal healthy skin.

Wherein the term "normal healthy skin" preferably refers to skin which is free from any infection.

Sometimes it might not be easy to distinguish between beneficial and harmful microbes because it might vary depending on circumstances. Therefore, for dysbiotic conditions like acne or atopic dermatitis, balancing means reducing or increasing the count of those microbes that have significantly changed in a condition as against healthy state. Such change could either be an abnormal increase or an abnormal decrease in their count.

A microbe can be described as a tiny living organism, such as bacterium, fungus, or virus. A microbiome or microbiota refers collectively to all the microbes on or in the human body. In other words, a microbiome is a community of microbes. A balanced microbiota containing diversity of organisms helps to maintain health and is essential for human development, immunity, health and wellbeing. Each of our individual microbiomes adapts throughout our lifetime and people can achieve a healthy microbiome in different ways. One way to regulate microbiome balance is via boosting skin's own mechanisms of innate immunity, e.g. via boosting the activity of antimicrobial lipids or peptides.

Acne, also known as Acne vulgaris, is a common skin condition that affects nearly all adolescents and/or adults. It is observed that acne usually occurs in areas rich in sebaceous glands like the face, neck and back. *Cutibacterium acne* (*C. acnes*) is thought to play a causative role. It is a bacterium.

Acne can be treated in variety of ways. Most treatments take several weeks to months before a noticeable change is seen. Benzoyl peroxide has an antibacterial effect has been used for mild cases of comedones and is also believed to prevent formation of other comedones. In severe cases, antibiotics like tetracycline, erythromycin and clindamycin are used. Antibiotics are believed to work by several mechanisms, the most important being the ability to bring about a decrease in the number of bacteria in and around the follicles. They are also thought to reduce the irritating chemicals produced by the white blood cells in the sebum, thereby reducing the inflammatory response.

According to one aspect of the present invention, skin means healthy skin. Such skin is non-dysbiotic skin.

Alternatively, skin is dysbiotic skin which is dry skin or has at least one of acne, atopic dermatitis, dandruff, melasma or is pollution-exposed skin. It is particularly preferred that dysbiotic skin is the skin which has acne.

Dandruff is a common scalp condition, characterized by excessive flaking and itch. It is generally accepted that the presence of dandruff is associated with changes in microbe ecology, altered lipid composition, inflammation and abnormal epidermal barrier function. The *Malassezia* yeast has long been considered as one of the main microbial drivers for dandruff. Severity of dandruff is generally associated with an increased abundance of *Malassezia restricta.* In addition, a trend towards an increase in the relative proportions of *Staphylococcus* to *Cutibacterium* is observed as dandruff scores increase, suggesting an imbalanced microbial ecology in the dandruff-affected scalp.

On the other hand, exposure of our skin to some pollutants may activate cutaneous stress as the pollutants could react with the skin and penetrate through the skin barrier and cause oxidative stress and inflammation by reacting with proteins, lipids and DNA molecules. Such exposure could manifest itself in the form of disorders and cosmetic conditions such as xerotic skin, sensitive skin and signs of premature or accelerated aging, such as wrinkles, abnormal pigmentation and dry skin. It is believed that certain pollutants may also cause acne, eczema and rashes.

Prolonged and repetitive exposure to pollutants, such as stressors like PM2.5 may impair the natural defense mechanisms of our skin leading to dysbiotic conditions like acne or dry skin. Moreover, some pollutants (e.g., ozone) can induce damage via signal transduction mechanism even when there is no percutaneous penetration into deeper layers of the skin.

In accordance with the present invention it is preferred that the microbes are bacteria. Alternatively, the microbes are at least one of fungi, protozoans or viruses.

Without wishing to be bound by theory it is believed that balanced microbiota of skin, is a result of synergy of thymol or terpineol or an analogue of thymol or terpineol in the topical composition with the host defense mechanism.

Preferably the at least one genus of harmful microbes is *Staphylococcus.* It is further preferred that upon use of the present invention, total count of bacteria belonging to genus *Staphylococcus* is reduced to the level normally indicative of healthy skin. Such levels are within the domain knowledge of persons skilled in the art of cosmetics and pharmaceutical products.

Further preferably the harmful microbes or at least one genus of microbes that exhibit abnormal growth further include bacteria belonging to at least one of the genus *Corynebacterium* or *Cutibacterium.*

It is particularly preferred that beneficial microbes or the at least one genus of microbes whose numbers have abnormally reduced belong to the genus *Streptoccocus, Moraxella* or *Deinococcus.*

### The Composition

The composition preferably comprises 0.02 to 5%, more preferably 0.03 to 1%, still more preferably 0.03 to 0.4% by weight thymol. Thymol may be used in purified form. Alternatively, thyme oil or thyme extract comprising thymol may be used instead of thymol while ensuring that amount of thymol contained therein is sufficient and efficacious. Thyme oil or thyme extracts are commercially available from a variety of local and global suppliers.

The composition preferably comprises 0.02 to 1%, still more preferably 0.03 to 0.4% by weight of the composition of terpineol. It may be used in purified form. The terpineol is preferably selected from alpha-terpineol, beta-terpineol, gamma-terpineol or a mixture thereof. It is particularly preferred that the terpineol is alpha-terpineol. Terpineol may be used in purified form. Alternatively, pine oil comprising terpineol may be added to the antimicrobial composition while ensuring required concentration/amount of terpineol in the composition.

The composition comprises 0.01 to 5.0 wt% each of thymol and terpineol. More preferably the composition comprises thymol and terpineol in individual amounts such that their total amount is 0.05 to 5.0 wt% by weight of the composition. Without wishing to be bound by theory, it is believed that the synergistic mixture of thymol and terpineol acts as antimicrobial agent to impede the cellular function of the targeted microbes.

It is preferred that the topical composition is a cosmetic composition. Alternatively, the topical composition is a medicament or a pharmaceutical composition.

The cosmetic composition is preferably in the form of a wash-off or a leave-on composition, more preferably a leave-on composition. Alternatively, it is a wash-off cosmetic composition, more preferably a facewash composition comprising 0.01 to 5.0 wt% each of thymol and terpineol.

Leave-on composition preferably means a composition which is not required to be removed or washed off from the human body after the application of the composition. When the composition is in the form of a leave-on composition, the composition is preferably a deodorant (stick, roll-on or spray), a hand sanitizer, a body lotion, a skin cream or body spray. Leave-on compositions are to be distinguished from compositions which are applied to the skin and subsequently removed, either by washing, rinsing, wiping, or the like either soon after or during the application of the product. Surfactants typically used for rinse-off compositions have physico-chemical properties giving them the ability to generate foam/lather in-use with ease of rinse; they can consist of mixtures of anionic, cationic, amphoteric, and nonionic surfactants.

The topical composition is preferably in the form of emulsions, which may be oil-in-water, or water-in-oil. In some embodiments, the skin care compositions are oil-in-water emulsions. Another format is a cream, including one which has a vanishing cream base. Vanishing cream base is one which comprises 5 to 40 wt% fatty acid and 0.1 to 20 wt% soap. In some embodiments, in such creams, the fatty acid is substantially a mixture of stearic acid and palmitic acid and the soap is the potassium salt of the fatty acid mixture, although other counterions and mixtures thereof can be used. The fatty acid in vanishing cream base is often prepared using hystric acid which is substantially (generally 90 to 95 percent) a mixture of stearic acid and palmitic acid. A typical hystric acid comprises 52 to 55 percent palmitic acid and 45 to 48 percent stearic acid of the total palmitic-stearic mixture. Thus, inclusion of hystric acid and its soap to prepare the vanishing cream base is within the scope of the present invention. In some embodiments, the skin care composition comprises higher than 7 percent, or higher than 10 percent, or higher than 12 percent fatty acid.

Alternatively, the topical composition is formulated as a single use personal care towelette product.

In some embodiments, the composition is non-solid. As used herein, the term "non-solid" means that the viscosity of the compositions, e.g. as measured using a Brookfield DV-I + viscometer (20RPM, RV6, 30 seconds, 20^{S}C). In some embodiments, the viscosity is in the range of from 1 Pas to 500Pas, alternatively from 1 Pas to 200Pas, alternatively from 2Pas to 100Pas, alternatively from 3Pas to 50Pas, at 20 degrees centigrade. In some embodiments, anionic surfactants are present in the leave-on skin care composition in an amount of at most 5 percent by weight of the composition, alternatively from 0.01 percent to 4 percent by weight of the composition, alternatively from 0.01 percent to 3 percent by weight of the composition, alternatively from 0.01 percent to 2 percent by weight of the composition, alternatively substantially absent (less than 1 percent, or less than 0.1 percent, or less than 0.01 percent). In some embodiments, the total level of surfactant in the skin care compositions is no more than 10 percent, alternatively below 8 percent, alternatively at most 5 percent.

If the composition is in the form of a deodorant, the composition may preferably comprise a conventional deodorant base as the cosmetically acceptable base. By a deodorant is meant a product in the stick, roll-on, or propellant medium which is used for personal deodorant benefit e.g. application in axilla, the under-arm area, which may or may not contain anti-perspirant actives. Deodorant compositions can generally be in the form of firm solids, soft solids, gels, creams, and liquids and are dispensed using applicators appropriate to the physical characteristics of the composition. Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95 wt% and in many instances from 40 to 80 wt%. Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly comprise water and/or a mono or polyhydric alcohol or water-miscible homologue. Polyhydric alcohols commonly comprise ethylene or propylene glycol, or a homologue can be employed such as diethylene glycol. Other than this suitable other vehicle and component used for deodorant composition can be added.

Wash-off composition preferably means a composition which is intended/required to be removed from the body by washing with solvent preferably water after the application of said composition like e.g. a shampoo, a hand wash composition and a face wash composition. In case of wash-off compositions, a cosmetically acceptable base preferably further comprises a surfactant.

For example, if the composition is in the form of a shampoo, in addition to water, the cosmetically acceptable base preferably comprises an anionic surfactant e.g. an alkyl sulphate and/or ethoxylated alkyl sulfate surfactant. These anionic surfactants are preferably present at a level of from 1 to 20 wt%, more preferably from 2 to 16 wt%, even more preferably from 3 to 16 wt%. Preferred alkyl sulfates are C8 to 18 alkyl sulfates, more preferably C12 to 18 alkyl sulfates, preferably in the form of a salt with a solubilizing cation such as sodium, potassium, ammonium or substituted ammonium.

It is preferred that the topical composition comprises a cosmetically acceptable base. Examples of ingredients that may be used as cosmetically acceptable base includes water, fatty acids, soaps (salts of fatty acids), alcohols and mixtures thereof.

In some embodiments, the skin care compositions of the present invention also include a cosmetically acceptable carrier. In some embodiments, where the personal care composition is a skin care composition, the cosmetically acceptable carrier is a lipophilic carrier that is liquid at temperatures up to 40°C.

The amount of the cosmetically acceptable carrier may vary in the skin care compositions according to some embodiments of the present invention. In some embodiments, the amount of the cosmetically acceptable carrier in a skin care composition may range from 1 to 99.9 percent by weight of the composition. Alternatively, the amount of the cosmetically acceptable carrier in a skin care composition may range from 70 percent to 95 percent by weight of the composition. Alternatively, the amount of the cosmetically acceptable carrier may range from 80 percent to 90 percent by weight of the composition. Alternatively, the amount of the cosmetically acceptable carrier may range from 5 to 99.9 percent by weight of the composition. Alternatively, the amount of the cosmetically acceptable carrier may range from 10 to 99.9 percent by weight of the composition. Alternatively, the amount of the cosmetically acceptable carrier may range from 15 to 99.9 percent by weight of the composition.

Cosmetically acceptable carriers suitable for the compositions include water, emollients, fatty acids, fatty alcohols, thickeners and combinations thereof.

In some embodiments, the cosmetically acceptable carrier for skin care compositions are aqueous and include water and oil emulsions of the W/O or O/W type or multiple emulsions of the W/O/W type. Water, when present in the compositions may be in amounts ranging from 5 percent to 95 percent by weight of the skin care composition. Alternatively, the water may be present in amounts ranging from 20 percent to 70 percent by weight of the skin care composition.

In some embodiments the cosmetically acceptable carrier is an emollient material. The emollient material may be in the form of silicone oils, natural or synthetic esters, hydrocarbons, alcohols and fatty acids. Amounts of the emollient material may range from 0.1 percent to 95 percent by weight of the composition.

Silicone oils may be volatile silicone oils, or non-volatile silicone oils. The term "volatile" as used herein refers to those materials which have a measurable vapor pressure at ambient temperature.

Volatile silicone oils suitable for a skin care composition according to some embodiments of the present invention may be cyclic (cyclomethicone) or linear polydimethylsiloxanes containing from 3 to 9 silicon atoms. In one embodiment, the linear polydimethylsiloxanes 5 to 6, silicon atoms.

Non-volatile silicone oils suitable for a composition include polyalkyl siloxanes, polyalkylaryl siloxanes and polyether siloxane copolymers. The non-volatile polyalkyl siloxanes useful herein include, for example, polydimethyl siloxanes with viscosities of from 5 x 10⁶ to 0.1 m²/s at 25°C. In some embodiments, the non-volatile silicone oils suitable for a skin care composition are polydimethyl siloxanes having viscosities from 1 x 10⁵ to 4 x 10⁴ m²/s at 25°C. Another class of non-volatile silicone oils suitable for a skin care composition are emulsifying and non-emulsifying silicone elastomers, such as, for example, Dimethicone/Vinyl Dimethicone Crosspolymer available as Dow Corning 9040, General Electric SFE 839, and Shin-Etsu KSG-18.

Another class of non-volatile silicone oils suitable for a skin care composition are silicone waxes such as, for example, Silwax WS-L (Dimethicone Copolyol Laurate) may also be useful.

Ester emollients may include alkyl esters of saturated fatty acids having 10 to 24 carbon atoms. Examples include, but are not limited to, behenyl neopentanoate, isononyl isonanonoate, isopropyl myristate and octyl stearate. In another example, ester emollients suitable for a skin care composition include ether-esters such as fatty acid esters of ethoxylated saturated fatty alcohols.

In another example, suitable ester emollients include polyhydric alcohol esters. Examples include, but are not limited to ethylene glycol mono and di-fatty acid esters, diethylene glycol mono- and di-fatty acid esters, polyethylene glycol (200- 6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl monostearate, 1,3- butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of Ci -C30 alcohols. In another example, ester emollients suitable for a skin care composition according to some embodiments of the present invention include wax esters such as, for example, beeswax, spermaceti wax and tribehenin wax.

In some skin care compositions natural ester emollients are based upon mono-, di- and tri- glycerides. Representative glycerides include, but are not limited to, sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof.

Some skin care compositions may comprise suitable hydrocarbons including, but not limited to, petrolatum, mineral oil, C11 to C13 isoparaffins, polybutenes and especially isohexadecane, available commercially as Permethyl 101 A from Presperse Inc. Fatty acids having from 10 to 30 carbon atoms may also be suitable. Illustrative of this category are pelargonic, lauric, myristic, palmitic, stearic, isostearic, oleic, linoleic, linolenic, hydroxystearic and behenic acids and mixtures thereof.

In some embodiments of skin care compositions the cosmetically acceptable carrier is fatty alcohols having from 10 to 30 carbon atoms. Suitable fatty alcohols include, but are not limited to, stearyl alcohol, lauryl alcohol, myristyl alcohol, oleyl alcohol and cetyl alcohol, or mixtures thereof.

Thickeners can be utilized as part of the cosmetically acceptable carrier of skin care compositions. Typical thickeners include crosslinked acrylates (e.g. Carbopol 982(R)), hydrophobically-modified acrylates (e.g. Carbopol 1382(R)), polyacrylamides (e.g. Sepigel 305(R)), acryloylmethylpropane sulfonic acid/salt polymers and copolymers (e.g. Aristoflex HMB(R) and AVC(R)), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for skin care compositions according to the present invention include, but are not limited to, guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum(R)).

Amounts of the thickener may range from 0.0001 to 10 percent, alternatively from 0.001 to 5 percent.

In some embodiments, the skin care composition contains a surfactant. The total concentration of the surfactant when present may range from 0.1 percent to 90 percent, alternatively from 0.1 percent to 80 percent. The amount of surfactant is dependent on a variety of factors, including, but not limited to, the type of personal care product. The surfactant is selected from the group consisting of anionic, nonionic, cationic and amphoteric actives. In some embodiments, nonionic surfactants are those with a C10-C20 fatty alcohol or acid hydrophobe condensed with from 2 to 100 moles of ethylene oxide or propylene oxide per mole of hydrophobe; C2-C10 alkyl phenols condensed with from 2 to 20 moles of alkylene oxide; mono- and di-fatty acid esters of ethylene glycol; fatty acid monoglyceride; sorbitan, mono- and di- C8-C20 fatty acids; and polyoxyethylene sorbitan as well as combinations thereof. In some embodiments, the non-ionic surfactant is selected from the group consisting of alkyl polyglycosides, saccharide fatty amides (e.g. methyl gluconamides) and trialkylamine oxides.

Amphoteric surfactants suitable in skin care compositions according to some embodiments of the present invention include cocoamidopropyl betaine, C12-C20 trialkyl betaines, sodium lauroamphoacetate, and sodium laurodiamphoacetate.

Anionic surfactants suitable in skin care compositions according to some embodiments of the present invention include soap, alkyl ether sulfates and sulfonates, alkyl sulfates and sulfonates, alkylbenzene sulfonates, alkyl and dialkyl sulfosuccinates, C8-C20 acyl isethionates, C8-C20 alkyl ether phosphates, C8-C20 sarcosinates, C8-C20 acyl lactylates, sulfoacetates and combinations thereof. Anionic surfactants are irritating to the skin, however, and skin care compositions of the invention are preferably devoid of anionic surfactants, i.e. contain less than 1 percent, and preferably less than 0.5 percent of the anionic surfactant.

In some embodiments, the skin care composition also includes 0.01 percent to 2 percent of a rheology modifier. In some embodiments, the rheology modifier is selected from the group consisting of silica such as fumed silica or hydrophilic silicas and clays such as magnesium aluminum silicate, betonites, hectorite, laponite, and mixtures thereof.

In some embodiments, the cosmetic composition, and especially a skin care composition contains sunscreen. The UV-B sunscreen oil may be selected from the class of cinnamic acid, salicylic acid, diphenyl acrylic acid, or derivatives thereof. The UV-B sunscreen oil may include one or more of octyl salicylate, 3,3,5-trimethylcyclohexyl 2-hydroxybenzoate, ethylhexyl salicylate, 2-ethylhexyl 2-cyano-3,3-diphenyl-2-propenoate, or 2-ethylhexyl- 4-methoxycinnamate (also known as octyl methoxycinnamate or "OMC"). Such UV-B sunscreen oils are typically commercially available, such as Octisalate^{™} (octyl salicylate), Homosalate^{™} (3,3,5-trimethyleyclohexyl 2-hydroxybenzoate), NeoHeliopan^{™} (a range of organic UV filters including OMC (Neo Heliopan AV^{™}) and ethylhexyl salicylate (Neo Heliopan OS^{™})), Octocrylene^{™} and Milestab 3039^{™} (2- ethylhexyl-2-cyano-3,3-diphenyl-2-propenoate) or Parsol MCX^{™} (2-ethylhexyl-4- methoxycinnamate). The amount of UV-B sunscreen oil in the personal care composition may be about 0.1 wt percent to about 20 wt percent.

The personal care composition may further include about 0.1 wt percent to about 10 wt percent of a UV-A sunscreen oil. The personal care compositions of the present technology that incorporate a UV-A sunscreen oil exhibit a significantly higher UVAPF when compared to compositions lacking the cyclocarboxylic acid. The UV-A sunscreen oil may include one or more of 4-t-butyl-4'- methoxydibenzoylmethane ("avobenzone"), 2-methyldibenzoylmethane, 4-methyl- dibenzoyl-ethane, 4-isopropyldibenzoyl-methane, 4-tert-butyldibenzoylmethane, 2,4- dimethyldibenzoylmethane, 2,5-dimethyldibenzoylmethane, 4,4'- diisopropyldibenzoylmethane, 2-methyl-5-isopropyl-4'-methoxy-dibenzoylmethane, 2- methyl-5-tert-butyl-4'-methoxy-dibenzoylmethane, 2,4-dimethyl-4'- methoxydibenzoylmethane, 2,6-dimehyl-4-tert-butyl-4'methoxydibenzoylmethane, diethylaminohydroxybenzoyl hexyl benzoate, ecamsule, or methyl anthranilate. The amount of UV-A sunscreen oil in the personal care composition may preferably be about 0.5 wt percent to about 7 wt percent, more preferably about 1 wt percent to about 5 wt percent.

The composition of any embodiment described herein preferably includes a skin lightening ingredient. Illustrative skin lightening ingredients include, but are not limited to, placental extract, lactic acid, niacinamide, arbutin, kojic acid, ferulic acid, hydroquinone, resorcinol, resorcinol derivatives (including 4-substituted resorcinols, such as especially 4-hexyl. 4-ethyl, 4-butyl, and/or 4-isopropyl resorcinols), dicarboxylic acids, 12-hydroxystearic acid ("12HSA"), and combinations of any two or more thereof. The skin lightening ingredient preferably includes a tyrosinase inhibitor to complement the melanogenesis inhibition activity of the substituted monoamines, such as kojic acid, hydroquinone and a 4-substituted resorcinol. Dicarboxylic acid skin lightening ingredients include those such as azelaic acid, sebacic acid and oxalic acid.

The amount of skin lightening ingredient may be about 0.1 wt percent to about 10 wt percent, or any range including and between these two values.

Anti-fungal agents suitable for inclusion in personal care compositions are well known to one of skill in the art. Examples include, but are not limited to, climbazole, ketoconazole, fluconazole, clotrimazole, miconazole, econazole, etaconazole, terbinafine, salts of any one or more of these (e.g., hydrochloride salts), zinc pyrithione, selenium disulfide, and combinations of any two or more thereof. Amounts of these materials may range from about 0.000001 wt percent to about 10 wt percent of the personal care composition,
The personal care composition may further include about 0.1 wt percent to about 8 wt percent of a film forming polymer. Such film-forming polymers include, but are not limited to, polyalkyleneoxy terminated polyamides (e.g., INCI name: Polyamide-3, Polyamide-4), polyether polyamides (e.g., INCI name: Polyamide-6), mixed acid terminated polyamides (e.g., INCI name: Polyamide-7), and ester terminated poly(ester-amides) (e.g., INCI name: Polyamide-8). Such film forming polymers may be synthesized or are available commercially, such as under the Sylvaclear^{™} line of products by Arizona Chemical Company, LLC and the OleoCraft^{™} line of products by Croda International PLC. Film-forming polymers also include, but are not limited to, the INCI named Polyester-5 (e.g., Eastman AQ^{™} 38S Polymer), PPG-17/IPDI/DMPA Copolymer (e.g., Avalure^{™} UR 450 Polymer), Acrylates Copolymer (e.g., Avalure^{™} AC 120 Polymer), and polysaccharides such as Xilogel (tamarin gum), lotus bean gums, tara gum, beta glucan, pullulan, carboxymethyl cellulose, hydroxypropyl cellulose, sodium alginate, potato starch, carrageenan.

Further ingredients useful in skin care compositions herein may be selected from any and all: skin conditioning agents, skin feel mildness agents, suspending agents, auxiliary thickening agents, viscosity control agents, dispersants, solubilizing/clarifying agents, stabilizers, opacifiers/pearlescent agents, chelating/sequestering agents, hydrotropes, bactericides/fungicides, antioxidants, pH control agents, buffering agents, colorants and perfumes/fragrances, water, other optional ingredients (auxiliary agents) and the like. The compositions of the present invention can also be optionally, incorporated into a water insoluble substrate for application to the skin such as in the form of a treated wipe. Preservatives can be incorporated into the personal care compositions according to some embodiments of the present invention to protect against the growth of potentially harmful microorganisms. Suitable preservatives for personal care compositions according to some embodiments of the present invention include, but are not limited to alkyl esters of para-hydroxybenzoic acid. Other suitable preservatives include hydantoin derivatives, propionate salts, and a variety of quaternary ammonium compounds. Other suitable preservatives include 1,2-alkane diols (e.g. 1,2-octane diol), phenoxyethanol, methyl paraben, propyl paraben, imidazolidinyl urea, sodium dehydroacetate and benzyl alcohol.

The colorants, opacifiers or abrasives may be included at a concentration from 0.05 percent to 5 percent, alternatively between 0.1 percent and 3 percent by weight of the composition. In some embodiments, the personal care product of the present invention may also include a pepdtide, such as, for example, the commercially available pentapeptide derivative- Matrixyl^{™}, which is commercially available from Sederma, France. In another example, in some embodiments, the personal care product of the present invention may also include Carnosine.

Preferably, the composition is formulated in the form of a powder, flake, lotion, cream, gel or mousse. More preferably, the composition is formulated in the form of cream or lotion and most preferably in the form of cream. The composition is preferably of leave-on type. The packaging for the composition of this invention can be a patch, bottle, tube, roll-ball applicator, propellant driven aerosol device, squeeze container or lidded jar.

The present invention now will be demonstrated by way of following non-limiting examples.

### Examples

### Example 1

Acne vulgaris is a multifactorial skin disorder. While proliferation of *Cutibacterium acnes* is historically considered to play a contributory role, recent advances in sequencing technology reveal that acne is associated with an altered microbiome profile involving multiple taxa that can be restored through appropriate interventions.

A double-blind, IRB approved, clinical study was conducted with 30-acne and 30 non-acne volunteers aged 18 to 30 years. The volunteers were given a standard facewash containing thymol and terpineol (thymol 0.1%, terpineol 0.25%) to use twice daily for four weeks while the non-acne control volunteers were given a marketed non anti-acne mild face cleanser which was devoid of thymol as well as terpineol and neither did it contain any analogue of either of them.

Facial microbiome/microbiota samples were collected using a cup scrub method, at baseline and 4-week after product usage. Microbial DNA was extracted from the samples using DNA extraction kit (Qiagen, DNeasy Blood & Tissue kit, 69506) following the manufacturer's instructions. The microbiome profile of 364 samples was characterized by 16s amplicon sequencing of the V1-V3 region on Illumina Miseq PE300 platform. Data analysis is performed using QIIME pipeline as described (Scientific Reports (7), 43344 (2017)). After quality processing, 3764 OTUs were found, which were classified into 24 phyla and 634 genera level taxa. Five phyla were dominant that comprised >1% (mean value) of the total population: *Actinobacteria, Firmicutes, Proteobacteria, Bacteroidetes* and *Deinococcus-Thermus.*

Eight predominant genera (mean value of relative abundance > 1%) were *Cutibacterium, Staphylococcus, Corynebacterium, Streptoccus, Moraxella, Deinococcus*, *Methylobacterium* and *Sphingomonas.*

Statistical analyses were performed on the table of counts at phylum and genus level. Analysis of Kruskal-Wallis was conducted to determine the statistical significance based on taxonomic profile or predicted functional pathway.

The data in Table 1 indicates that acne lesions are associated with altered microbiome, and a facewash, i.e. a topical composition which is a cosmetic composition that comprises thymol as well as terpineol can relieve acne symptoms and can balance the microbiota of amenable skin towards a level closer to health.

In Table 1 is shown the relative abundance of dominant genera of the skin microbiota of healthy baseline v/s acne baseline and 4-weeks after cosmetic treatment with a topical facewash comprising thymol and terpineol.

**Table 1**

| Genus | Relative abundance (%) | | |
|---|---|---|---|
| | Healthy skin | Acne skin | Acne skin 4-weeks later |
| *Staphylococcus* | 30.9 | 39.1* | 28.7 |
| *Cutibacterium* | 23.3 | 24.5 | 22.5 |
| | | | |
| *Streptococcus* | 2.3 | 1.3* | 1.6 |
| *Moraxella* | 1.5 | 0.7* | 2.2* |
| *Deinococcus* | 1.1 | 0.2* | 0.6* |
| *Methylobacterium* | 0.5 | 0.2 | 0.6 |
| *Sphingomonas* | 0.7 | 0.3 | 1.1 |

| | | | |
|---|---|---|---|
| *Microbial abundance significantly different from the healthy baseline (P<0.05) | | | |

In Table 2 is shown the relative abundance of dominant phyla of the skin microbiome of healthy baseline vs acne baseline and 4-week after cosmetic treatment with the face wash.

**Table 2**

| Phylum | Relative abundance (%) | | |
|---|---|---|---|
| | Healthy skin | Acne skin | Acne skin 4-weeks later |
| *Actinobacteria* | 35.7 | 38.3 | 37.5 |
| *Bacteroidetes* | 19.5 | 15.7* | 17.9 |
| *Firmicutes* | 35.6 | 42.4* | 32.3 |
| *Proteobacteria* | 24.0 | 16.7* | 26.6 |
| *Deinococcus-Thermus* | 1.1 | 0.2* | 0.6 |

| | | | |
|---|---|---|---|
| *Microbial abundance significantly different from the healthy baseline (P<0.05) | | | |

The relative abundancy profile of the predominant bacteria genera indicate there was microbiome dysbiosis in acne, with significantly higher genera levels of *Staphylococcus* and *Cutibacterium* and lower levels of *Streptoccoccus*, *Moraxella* and *Deinococcus,* as compared to the non-acne skin of healthy subjects. After intervention of the facewash comprising thymol and terpineol, most of the predominant genera except *Moraxella* and *Deinococcus* were fully restored to the normal level that showed no difference from the healthy baseline.

Relative abundance profile of the predominant bacteria phyla indicated microbiome dysbiosis in acne, with significantly higher phyla levels of *Firmicutes* and lower levels of *Bacteroidetes*, *Proteobacteria*, and *Deinococcus-Thermus*, as compared to the non-acne skin of the healthy subjects. After intervention of a facewash comprising thymol and terpineol, all the predominant phyla were fully restored to the normal level that showed no difference from the healthy baseline.

In conclusion, a facewash comprising thymol and terpineol balanced microbiome composition in acne skin by selectively reducing microbial count of *Staphylococcus* and *Cutibacterium* while selectively increasing the microbial count of *Streptoccoccus*, *Moraxella* and *Deinococcus.*

## Claims

1. Non-therapeutic use of thymol and terpineol for balancing microbiota of healthy non-dysbiotic skin, where balancing means selectively reducing microbial count of at least one genus of microbes belong to *Staphylococcus* or *Cutibacterium,* while selectively increasing microbial count of at least one genus of microbes belong to *Streptoccocus*, *Moraxella or Deinococcus.*

2. A non-therapeutic method of balancing microbiota of healthy non-dysbiotic skin comprising a step of applying thereto thymol and terpineol in a topical cosmetic composition, where balancing means selectively reducing microbial count of at least one genus of microbes belong to *Staphylococcus or Cutibacterium*, while selectively increasing microbial count of at least one genus of microbes belong to *Streptoccocus*, *Moraxella or Deinococcus.*

3. A method as claimed in claim 2 to wherein said topical cosmetic composition comprises 0.01 to 5 wt% of each of thymol and terpineol.

4. A topical composition comprising thymol and terpineol for use in balancing microbiota of dysbiotic skin which is dry skin or has at least one of acne, atopic dermatitis, dandruff, melasma or is pollution-exposed skin, where balancing means selectively reducing microbial count of at least one genus of microbes *Staphylococcus or Cutibacterium*, while selectively increasing microbial count of at least one genus of microbes belong to *Streptoccocus*, *Moraxella or Deinococcus.*

5. A topical composition for use according to claim 5, wherein said dysbiotic skin is skin which has acne.

## Patentansprüche

1. Nicht-therapeutische Verwendung von Thymol und Terpineol zum Ausgleich der Mikrobiota gesunder, nicht dysbiotischer Haut, wobei Ausgleich die selektive Verringerung der Keimzahl mindestens einer Gattung von Mikroben, die zu Staphylococcus oder Cutibacterium gehören, bei gleichzeitiger selektiver Erhöhung der Keimzahl mindestens einer Gattung von Mikroben, die zu Streptococcus, Moraxella oder Deinococcus gehören, bedeutet.

2. Nicht-therapeutisches Verfahren zum Ausgleich der Mikrobiota gesunder, nicht dysbiotischer Haut, umfassend einen Schritt des Aufbringens von Thymol und Terpineol in einer topischen kosmetischen Zusammensetzung, wobei Ausgleich die selektive Verringerung der Keimzahl mindestens einer Gattung von Mikroben, die zu Staphylococcus oder Cutibacterium gehören, bei gleichzeitiger selektiver Erhöhung der Keimzahl mindestens einer Gattung von Mikroben, die zu Streptococcus, Moraxella oder Deinococcus gehören, bedeutet.

3. Verfahren nach Anspruch 2, wobei die topische kosmetische Zusammensetzung 0,01 bis 5 Gew.-% an jeweils Thymol und Terpineol umfasst.

4. Topische Zusammensetzung, die Thymol und Terpineol umfasst, zur Verwendung beim Ausgleich der Mikrobiota dysbiotischer Haut, die trockene Haut ist oder mindestens eines der Merkmale Akne, atopische Dermatitis, Schuppen, Melasma aufweist oder durch Umweltverschmutzung belastete Haut ist, wobei Ausgleich die selektive Verringerung der Keimzahl mindestens einer Gattung von Mikroben, die zu Staphylococcus oder Cutibacterium gehören, bei gleichzeitiger selektiver Erhöhung der Keimzahl mindestens einer Gattung von Mikroben, die zu Streptococcus, Moraxella oder Deinococcus gehören, bedeutet.

5. Topische Zusammensetzung zur Verwendung gemäß Anspruch 5, wobei die dysbiotische Haut eine Haut ist, die Akne aufweist.

## Revendications

1. Utilisation non thérapeutique de thymol et de terpinéol pour équilibrer le microbiote d'une peau saine non dysbiotique, où équilibrer signifie réduire sélectivement le compte microbien d'au moins un genre de microbes appartenant à *Staphylococcus* ou *Cutibacterium,* tout en augmentant sélectivement le compte microbien d'au moins un genre de microbes appartenant à *Streptococcus*, *Moraxella* ou *Deinococcus.*

2. Méthode non thérapeutique pour équilibrer le microbiote d'une peau saine non dysbiotique, comprenant une étape d'application à celle-ci de thymol et de terpinéol dans une composition cosmétique à usage topique, où équilibrer signifie réduire sélectivement le compte microbien d'au moins un genre de microbes appartenant à *Staphylococcus* ou *Cutibacterium,* tout en augmentant sélectivement le compte microbien d'au moins un genre de microbes appartenant à *Streptococcus*, *Moraxella* ou *Deinococcus.*

3. Méthode selon la revendication 2, dans laquelle ladite composition cosmétique à usage topique comprend 0,01 à 5 % en poids de chacun parmi le thymol et le terpinéol.

4. Composition à usage topique comprenant du thymol et du terpinéol pour une utilisation dans le but d'équilibrer le microbiote d'une peau dysbiotique qui est une peau sèche ou qui a au moins l'un parmi un acné, une dermatite atopique, des pellicules, un mélasme ou qui est une peau exposée à une pollution, où équilibrer signifie réduire sélectivement le compte microbien d'au moins un genre de microbes appartenant à *Staphylococcus* ou *Cutibacterium,* tout en augmentant sélectivement le compte microbien d'au moins un genre de microbes appartenant à *Streptococcus*, *Moraxella* ou *Deinococcus.*

5. Composition à usage topique pour une utilisation selon la revendication 5, dans laquelle ladite peau dysbiotique est une peau qui a de l'acné.
